# EUROPEAN PATENT SPECIFICATION

(11) **EP 0 859 597 B1**
(45) Date of publication and mention of the grant of the patent: **09.04.2003**
(21) Application number: 96933878.9
(22) Date of filing: 25.09.1996
(51) Int. Cl.: A61K 9/08, A61K 47/36, A61K 47/38

(54) **SUSTAINED RELEASE DELIVERY SYSTEM AND LONG ACTING NARCOTIC ANALGESICS AND ANTAGONISTS**
VERABREICHUNGSSYSTEM MIT VERZOEGERTER FREISETZUNG SOWIE LANGWIRKENDE NARKOTISCHE ANALGETIKA UND ANTAGONISTEN
SYSTEME D'ADMINISTRATION A LIBERATION PROLONGEE ET ANTAGONISTES ET ANALGESIQUES NARCOTIQUES A EFFET DE LONGUE DUREE

(30) Priority: 29.09.1995 US 536750; 18.01.1996 US 588272; 10.04.1996 US 630205
(43) Date of publication of application: 26.08.1998
(73) Proprietor: LAM Pharmaceutical Corporation, Lewiston New York 14092 (US)
(72) Inventor: DRIZEN, Alan, Downsview, Ontario M3H 5T98 (CA); ROTHBART, Peter, Toronto, Ontario M4R 1H5 (CA); NATH, Gary M., Bethesda, Maryland 20817 (US)
(74) Representative: Evens, Paul Jonathan
(86) International application number: US9615293
(87) International publication number: WO97011681

(56) References cited:
- EP-A- 0 516 026
- FR-A- 2 542 616
- US-A- 5 143 724
- US-A- 5 356 629
- US-A- 5 358 973

## Description

### BACKGROUND OF THE INVENTION

### 1. Field of the Invention

This invention relates to the preparation of a sustained release delivery system, and more particularly to a system using a polymer matrix containing a drug. The system is designed to administer effective levels of drugs over a sustained period of time when administered intramusculariy, epidurally or subcutaneously for the treatment of various disease states conditions. A particularly advantageous use of the system is the administration of a local anesthetic along the sheath of a nerve or muscle tissue to alleviate or ameliorate the effects of pain.

This invention also relates to the preparation of long acting analgesics, and more particularly to a water-soluble system for the intramuscular administration of a narcotic drug. The system is designed to administer effective levels of drugs over a sustained period of time when administered intramuscularly, for the treatment of pain and drug addiction.

### 2. Description of the Prior Art

Medications have been formulated to enable the administration of drugs to occur over a wide variety of paths, including instantaneous delivery by use of injectables, and sustained, controlled and extended release delivery by use of tablets, capsules, and particulate forms which enable release of the drug to be controlled by various means, such as by resistance of the structure's coating or composition against diffusion of the drug therethrough. These systems have all found wide applications for the delivery of drugs.

EP-A-0 516 026 discloses a sustained release hydrogel being composed of a PVA, a drug, and a hydrophilic polymer such as hyaluronic acid.

FR-A-2 542 616 discloses gels for topical application to the skin comprising a negatively charged polymer, an excipient such as hydroxyethylcellulose and an antibiotic.

US-A-5,143,724 discloses a sterile, injectable gel slurry comprising a glycosamino-glycan gel-phase and a polymer solution of a water-soluble polymer,

US-A-5, 358, 973 discloses a sterile composition comprising a drug, hyaluronic acid and dextran for use in surgical operations.

None of the known drug delivery systems, however, are able to administer effective therapeutic amounts of a drug for sustained periods of time, that is, longer than 24 to 48 hours. Actually, most delivery systems maintain effective dosages for from several hours to daily doses before requiring readministration. Such systems have not been found to be effective for the long term administration of drugs that require repetitive and continued use, except of course for selected patch treatments. Drugs that have been repeatedly administered for long term treatments include but are not limited to analgesics, antagonists and anesthetics for treating pain, steroids and hormone administration for maintenance, modification or alteration of body chemistry, metabolism and hormone balance and regulation, vitamin and mineral supplementation, and so forth. A delivery system is therefore needed which would permit the administration of therapeutically effective amounts of drugs to enable a continued and sustained release for at least 24 hours to several days.

Morphine and drugs having similar structures, especially opioids, have been used for pain relief, and primarily treatment of nociceptive pain, namely that due to irritation or damage of pain receptors in the skin or nearby tissues. Examples of such narcotics include morphine sulfate, Dilaudid, Demerol, codeine, Taliwin and Percocet.

The problems associated with narcotic use are manifold, but primarily relate to their relative short duration for pain relief, namely two to five hours with intramuscular injection, and secondly, they are addictive, especially when used in large doses. Tolerance and physical dependence on both natural and synthetic opioids develops rapidly; therapeutic doses taken regularly over a two or three day period can lead to some tolerance and dependence, and the user may show symptoms of withdrawal when the drug is discontinued. Furthermore, opioid drugs induce cross-tolerance and abusers may substitute one drug for another.

Currently, analgesics are used to treat various pain conditions in several ways.
(1) intramuscular or oral administration of morphine, Dilaudid or codeine with repeated injections every two to five hours;
(2) patient controlled analgesia where the patient operates an intravenous drip with control of the amount of drug by a computer delivery procedure where small amounts are administered on demand; and
(3) continuous epidural pump infusions where the analgesic is administered by a computerized pump through tubing into the epidural space and wherein the dose is continuously adjusted.

A delivery system is therefore needed which would permit the administration of therapeutically effective amounts of analgesic and antagonist drugs to enable a continued and sustained release for at least 12 hours to several days.

### SUMMARY OF THE INVENTION

The present invention relates to the formation of a long-acting drug composition for use in treating acute, or chronic conditions. More particularly, this invention relates to a sterilized, purified, solubilized or suspended drug composition, which comprises: a drug dispersed within a polymer matrix, solubilized or suspended in a polymer matrix, with or without the presence of a preservative. The polymer matrix is composed of a highly negatively charged polymer material (hyaluronic acid salts) and a non-ionic polymer selected from the group consisting of hydroxyethyl cellulose, hydroxypropyl cellulose, and mixtures thereof. Accordingly, there is provided in accordance with the present invention a sustained release delivery system as defined in claim 1 appended hereto, and to which reference should now be made. Other aspects and embodiments are defined in other claims appended hereto, and to which reference should also now be made.

Another embodiment of this invention involves the use of the system in the preparation of a long-acting medicament for the treatment of a condition in animals, which medicament is for injecting therapeutically effective dosages of a suspension or solution of a sterilized, purified, solubilized or suspended composition comprising a drug dispersed within a polymer matrix which is solubilized or suspended in a liquid medium. Preferably, one of the polymer materials has a mean average molecular weight below about 800,000, and the other polymer is a non-ionic cellulose derivative selected from hydroxymethyl cellulose, hydroxypropyl cellulose and mixtures thereof.

The present invention also relates to the formation of long-acting analgesic and antagonist composition for use in treating acute or chronic pain conditions and aiding in treating drug addiction. More particularly, this invention relates to an injectable narcotic drug solubilized in a liquid polymer matrix, with or without the presence of a preservative. The polymer matrix is composed of a salt of hyaluronic acid and a non-ionic polymer selected from the group consisting of hydroxyethyl cellulose, hydroxypropyl cellulose, and mixtures thereof.

Another embodiment of this invention involves the use of the system in the preparation of a long-acting medicament for the treatment of a condition in animals, which medicament is for intramuscularly injecting therapeutically effective doses (which may be less than the normal therapeutic dosage) of a solution of a narcotic solubilized drug within an aqueous liquid containing a polymer matrix. Preferably one of the polymer materials has a mean average molecular weight below about 800,000, and the other polymer is a non-ionic cellulose derivative selected from hydroxymethyl cellulose, hydroxypropyl cellulose and mixtures thereof.

An alternate embodiment relates to the use of the present formulations to treat drug addiction.

### DETAILED DESCRIPTION OF THE INVENTION

The present invention relates to the formation of a delivery system for administering a drug for a sustained period, and particularly to a polymer matrix useful for its treatment of acute, or chronic intractable pain and to the use therefor. The process involves the production and use of specialized compounds manufactured by using polymers of molecular weights below about 800,000 in a unique process for the creation of specially modified molecules to treat a variety of conditions. Specifically, the invention addresses a process for manufacturing a polymer matrix suspended or solubilized in water with various drugs. The polymers must be sterilizable and acceptable for animal, and human use. In this way, a suitable polymer system is formed as a matrix which is able to disperse a much lower molecular weight drug to form a solution or suspension of the active for subsequent use.

It has been found in conventional drug treatments that once a therapeutic dosage is used, the beneficial effect of such dosage routine wears off within several hours of its initial application; thus requiring repetitive treatment. This mechanism is common in all animal systems and involves biochemical pathways that have not yet been fully discovered or identified. One possibility for this action would involve the animals own immunoglobin system which may be responsible for identifying the presence of the chemical entity and systematically destroying it. Another may be the inherent instability of the chemical entity after its administration into the animal.

It has been unexpectedly discovered that an effective therapeutic level of a drug may be administered once over at least a 24 hour to several day interval and preferably over at least a 12 to 24 hour period when the drug is suspended or entrapped in a specially designed polymer matrix containing almost equal molar ratios of a negatively charged polymer selected from hyaluronic salts and a non-ionic polymer selected from hydroxyethyl cellulose, hydroxypropyl cellulose and mixtures thereof suspended or dissolved in water.

This system is believed to form a matrix which micro-encapsulates, suspends and/or entraps the active drug entity such that when it is administered it is slowly released into the systemic circulatory system or muscular tissue providing a sustained and prolonged drug release rate.

The molar ratio of the polymers present in the matrix is critical in this invention. It has been found that molar ratios of the negatively charged polymer to the non-ionic polymer must be from 1:0.5 to 2 and preferably from 1:0.8 to 1.5 and most preferably from 1:1 to 1.3. At ratios either higher or lower than these levels the resulting systems tend to sheer when being prepared and form unacceptable air pockets and bubbles. Furthermore, the solutions tend to separate and form distinct polymer layers.

At least one of the polymers used to form the matrix of this invention must be sufficiently negatively charged to aid in the dispersion, encapsulation or solubilization of the drug. Particularly preferred polymers have mean average molecular weights below about 800,000 and preferably molecular weights between about 500,000 to 800,000 have been found acceptable to form useable polymer matrixes. Polymers with mean average molecular weights between about 700,000 and 775,000 are most preferred. Polymers having molecular weights above about 800,000 form solid gels in solution and are unable to serve in an injectable system. Furthermore, the polymers must be sterilizable and be stable during sterilization so that the polymer does not loss molecular weight once formulated into the final injectable form.

Examples of compounds that may be used as a source of this molecular weight polymer are hyaluronic acid salts, with sodium hyaluronate being most preferred.

Hyaluronic acid (HA) occurs naturally in joint synovial fluid, where it plays a lubricating role, and may have biological activity as well. HA is a mucopolysaccharide, and may alternatively be referred to as a glycosaminoglycan. The repeating unit of the hyaluronic acid molecule is a disaccharide consisting of D-glucuronic acid and N-acetyl-D-glucosamine. Because hyaluronic acid possesses a negative charge at neutral pH, it is soluble in water, where it forms highly viscous solutions. The D-glucuronic acid unit and N-acetyl-D-glucosamine unit are bonded through a glycosidic, beta (1-3) linkage, while each disaccharide unit is bonded to the next disaccharide unit through a beta (1-5) linkage. The (beta 1-4) linkages may be broken through hydrolysis with the enzyme hyaluronidase.

A variety of substances, commonly referred to as hyaluronic acid, have been isolated by numerous methods from various tissue sources including umbilical cords, skin, vitreous humour, synovial fluid, tumors, haemolytic streptocci pigskin, rooster combs, and the walls of veins and arteries. It is also being synthesized artificially and by recombinant technology.

Conventional methods for obtaining hyaluronic acid results with a product having differing properties and a wide range of viscosities. U.S. Patent No. 2,585,546 to Hadian, discloses an example of a method for obtaining hyaluronic acid and which involves extracting acetone-washed umbilical cords with a dilute salt solution, acidifying the resulting extract, removing the clot so formed, precipitating some hyaluronic acid with protein from the acidified extract with ammonium sulfate, agitating the liquid with pyridine, precipitating another fraction highly contaminated with protein, followed by more ammonium sulfate which forces some pyridine out of solution along with the high viscosity hyaluronic acid. The hyaluronic acid collects at the interface between the two liquid phases and may be separated by filtration, centrifugation or other usual procedure. A modification of this process involves the fractionation of the acidic salt extract from umbilical cords with alcohol and ammonium sulfate. Alcohol is added to the acidic salt extract, and the resulting precipitate is removed. Solid ammonium sulfate is added to the liquid until saturation and the solution forms two phases with a precipitate of hyaluronic acid at the interface.

U.S. Patent No. 4,517,296 to Bracke et al, is directed to the preparation of hyaluronic acid in high yield from streptococcus bacteria by fermenting the bacteria under anaerobic conditions in a CO₂ enriched growth medium, separating the bacteria from the resulting broth and isolating the hyaluronic acid from the remaining constituents of the broth. Separation of the microorganisms from the hyaluronic acid is facilitated by killing the bacteria with trichloroacetic acid. After removal of the bacteria cells and concentration of the higher molecular weight fermentation products, the hyaluronic acid is isolated and purified by precipitation, resuspension and reprecipitation.

One particular fraction of hyaluronic acid (HA) that exhibits excellent matrix formation according to the present invention is hyaluronate sodium having a molecular weight of between 650,000 - 800,000, preferably 700,000 - 775,000 with a high degree of purity, 55-105% free, and preferably at least 98% pure, from contamination of related mucopolysaccharides. Furthermore, this hyaluronic acid has a sulphated ash content of less than 15% and a protein content of less than 5%. Examples of usable base salts include those safe for animal and human use, such as sodium, potassium, calcium, and magnesium salts or the like.

The negatively charged polymers are generally present in the system in amounts which enable a solution or solid gel to be formed. Generally, solutions are formed using amounts of about 0.1 to 2.0% by weight with amounts of about 1 to about 1.5% by weight being preferred for use as an injectable. Topical gel forms may be prepared with amounts of about 2.0% to about 3.0% by weight. A particularly preferred sodium HA concentration as an injectable is about 1.2% to about 1.4% by weight of the system and preferably 1.3%.

In addition to the negatively charged polymers, the polymer matrix must contain a non-ionic polymer which aids in retarding the rate of absorption of the active drug and delays or slows down an animals natural absorption of the negatively charged polymer. without the presence of this component, the drug would be rapidly absorbed, and sustained action of the active could not be achieved. The non-ionic polymers are cellulose derivatives selected from the group consisting of hydroxyethyl cellulose (HEC), hydroxypropyl cellulose and mixtures thereof. These particular polymers have been found to possess exceptional ability to form sustained release matrix formulations when used in combination with a negatively charged polymer. Such polymers are generally employed in amounts of about 0.1% to about 1.5% and preferably about 0.5% to about 1.4%. Amounts above about 1.0% or 1.4% result in the formation of a solid gel product when used with the negatively charged polymer. Amounts below about 0.1% have not been found suitable to prepare a storage stable solution or form a product that has sustained drug release.

A particularly preferred HEC concentration is about 1.2% to about 1.3% by weight of the system.

A wide variety of medicaments which are administered may be used in the delivery system according to this invention. These include drugs from all major categories, and without limitation, for example, anesthetics including those used in caudal, epidural, inhalation, injectable, retrobulbar, and spinal applications, such as bupivacaine and lidocaine; analgesics, such as acetaminophen, ibuprofen, fluriprofen, ketoprofen, voltaren (U.S. Patent No. 3,652,762), phenacetin and salicylamide; anti-inflammatories selected from the group consisting of naproxen and indomethacin; antihistamines, such as chlorpheniramine maleate, phenindamine tartrate, pyrilamine maleate, doxylamine succinate, phenyltoloxamine citrate, diphenhydramine hydrochloride, promethazine, brompheniramine maleate, dexbrompheniramine maleate, clemastine fumarate and triprolidine; antitussive selected from the group consisting of dextromethorphan hydrobromide and guaifenesin; expectorants such as guaifenesin; decongestants, such as phenylephrine hydrochloride, phenylpropanolamine hydrochloride, pseudoephedrine hydrochloride, ephedrine; antibiotics including amebicides, broad and medium spectrum, fungal medications, monobactams and viral agents and specifically including such as erythromycin, penicillin and cephalosporins and their derivatives; bronchodilators such as theophylline, albuterol and terbutaline; cardiovascular preparations such as diltiazem, propranolol, nifedepine and clonidine including alpha adrenoceptro agonist, alpha receptor blocking agent, alpha and beta receptor blocking agent, antiotensin converting enzyme inhibitors, beta blocking agents, calcium channel blocker, and cardiac glycosides; central nervous system drugs such as thioridazine, diazepam, meclizine, ergoloid mesylates, chlorpromazine, carbidopa and levodopa; metal salts such as potassium chloride and lithium carbonate; minerals selected from the group consisting of iron, chromium, molybdenum and potassium; immunomodulators; immunosuppressives; thyroid preparations such as synthetic thyroid hormone, and thyroxine sodium; steroids and hormones including ACTH, anabolics, androgen and estrogen combinations, androgens, corticoids and analgesics, estrogens, glucocorticoid, gonadotropin, gonadotropin releasing, human growth hormone, hypocalcemic, menotropins, parathyroid, progesterone, progestogen, progestogen and estrogen combinations, somatostatin-like compounds, urofollitropin, vasopressin, and others; and vitamins selected from water-soluble vitamins such as B complex, vitamin C, vitamin B12 and folic acid and veterinary formulations.

Particularly preferred dosage forms involve use of bupivacaine, lidocaine, vitamin B12, methyl prednisolone and adenosine-5-monophosphate sodium.

A wide variety of analgesics and antagonist drugs which are administered by injection may be used in the delivery system according to the invention.

One particular criteria of the drug is that they must be solubilized in the polymer matrix solution in order to be injected intramuscularly. Without limitation, this includes analgesics and antagonists that are relatively water-soluble, that is, soluble enough to be dissolved or suspended in the polymer matrix solution so that they may be administered by injection. Particularly preferred drugs include, without limitation, analgesics such as morphine and its salts, such as morphine sulfate, codeine, meperidine, methadone, propoxyphene, levophanol, hydromorphone, oxymorphone, oxycodone, as well as opioid antagonists and agonist-antagonists such as naloxone, naltyhexone, pentazocaine, butorphanol, nalbuyphine, and buspprenorphine. The equianalgesic doses of exemplary opioid analgesics for severe pain is set forth in TABLE I derived from the 16th Edition of the Merck Manual, page 1414. Besides these drugs, exemplary nonlimiting drugs also include:
- TALWIN®: (pentazocaine lactate) which chemically is 1,2,3,4,5,6-hexahydro-6, 11-dimethyl-3-(3-methyl-3 butenyl)-2,6-methano-3-benzanocin-8-ol lactate.
- DEMEROL®: (meperidine hydrochloride) which chemically is 1-methyl-4-phenylisonipecolate hydrochloride.
- Methadone Hydrochloride: which chemically is 3-heptanone, 6-(dimethylamnino)-4,4-diphenylhydrochloride.
- LEVO-DROMORAN®: also known as levorphanol tartrate.
- BUPROENEX®: (buprenorphine hydrochloride) which chemically is 17-(cyclopropylmethyl)-α-(1,1-dimethylethyl)-4, 5-epoxy-18, 19-dihydro-3-hydroxy-6-methoxy-α-methyl-6, 14-ethenomorphinan-7-methanol, hydrochloride [5α,7α(S)].
- MSIR®: (morphine sulfate) which chemically is 7,8 didehydro-4,5-α-epoxy-17-methyl-morphinian-3,6 α-diol sulfate (2:1)(salt) pentahydrate.
- DILAUDID® SUFENTA®: also known as hydromorphone hydrochloride. (sufentanil citrate) which chemically is N-[-4-(methyoxymethyl) (-1- [2-(2-thienyl) ethyl]-4-piperidinyl]-N-phenylpropanamide 2-hydroxy-1,2,3,-propanetricarboxylate.
- SUBLIMAZE®: (fentanyl citrate) which chemically is N-(1-phenethyl-4-piperidyl) propionanilide citrate.
- AFENTA®: (afentanil hydrochloride) which chemically is N-[1-[2-(4-ethyl -4,5-dihydro-5-oxo-1H-tetranol-1-yl) ethyl]-4- (methoxymethyl)-4-piperidinyl]-N-phenyl propanamide monohydrochloride.
- PERCOCET®: which is a combination of oxycodone hydrochloride and acetaminophen.
- NUMORPHAN®: (oxymorphone hydrochloride) which chemically is 4,5 α-Epoxy-3,14-dihydrox-17-methylmorphinon-6-one hydrochloride.

The solutions or suspensions of the present invention may be prepared in a variety of ways. For example, the polymers may be dissolved in water and purified either separately or jointly and then the active drug added to the system.

A particularly preferred procedure involves separately dissolving the nonionic polymer in water and centrifuging the material to form a solution and remove impurities. This may be conveniently done at rotation speeds of 2000 rpm for times of about 30 minutes to about two hours.

In contrast, the charged polymer may be blended and stirred in water until it is dissolved. This process must be done while avoiding the formation of bubbles and while freeing the polymer of its electrostatic activity. Furthermore, the molecular weight of the polymer must not be significantly changed during processing and as such mild process conditions are required. Processing conditions of 400 - 600 rpm for durations of 16 - 24 hours have been found acceptable to produce stable solutions or gels of the charged polymer.

Conventional pharmaceutically acceptable emulsifiers, suspending agents, antioxidants (such as sodium meta-bisulfate) and preservatives may then be added to this system. Once all the components are blended together, such as by mixing 400 - 600 rpm for one to four hours, the system is filled into tubes and sterilized. The resulting system is a clear solution which is storage stable for several years.

The drug may then be added to the homogenous solution or separately dissolved or disbursed in water. Emulsifiers, suspending agents and preservatives may then be added to this system. Once all the components are blended together, 400 - 600 rpm for 1 to 4 hours, the system is filled into tubes and sterilized. The resulting system is a clear solution which is storage stable for several years.

When gels are prepared, the resulting system is also a clear gel or opaque which can be filled into tubes or containers and stored for future use. The formulations may be used topically and also contain conventional pharmaceutically acceptable excipients well known to those skilled in the art, such as surfactants, suspending agents, emulsifiers osmotic enhancers, extenders and dilutants, pH modifiers as well as fragrances, colors, flavors and other additives.

As indicated above, the drugs may be blended with the aqueous polymer matrix at the time of manufacture or simply mixed together, such as by shaking, at the time of use. As such, the drug when in the form of a water-soluble solid is simply diluted with sterilized water or polymer matrix solution, dissolved and immediately injected into the patient. Alternatively, previously prepared solutions of the drug are blended with the polymer matrix solution, such as in a syringe or the cartridges already containing the solutions, and then injected into the patient. These procedures enable the use of commercially prepared narcotic dosage forms without need fcr separate processing, handling or storage procedures.

The present long acting narcotic delivery system enables the use of reduced levels of narcotics to be administered over a given period of time which does not cause the highs and lows associated with the two to five hour doses. It also is very likely to decrease the potential for drug addiction by reducing the level of doses required to reduce the pain. In this regard, it has been found that use of a single or multiple amount of a single dose (of a conventional dosage) administered once every 24 hours when mixed with the aqueous polymer matrix will achieve the same effect as six to ten conventional doses administered every two to five hours of duration.

The injection of a single daily dose is extremely easy and safe to administer and obviates the need for machinery maintenance (pump devices), continuous nursing care monitoring and dosage recalculation associated with epidural pumps.

The dosage system can be formed with or without the use of pharmaceutically acceptable preservatives. A significant advantage of the dosage form of the present system relates to its ability to allow the drug to slowly diffuse through tissue when injected intramuscularly, thus allowing for an effective therapeutic dose to be present for many hours.

In this regard, it should be noted that reference to therapeutically effective dose does not necessarily relate to conventional dosage levels, but does relate to drug levels that achieve an effective therapeutic level at the dose emolcyed, which may be the same level but not at the same frequency of administration. This thus not only significantly reduces the number of doses required to achieve the same effect, it reduces costs, maintenance and health hazards associated with conventional treatment therapies. Additionally, it results in immediate and continued drug release for long periods of time spanning at least 18 hours to even days.

Doses may vary from patient to patient depending on the type and severity of the condition being treated and the drug being administered. Generally, doses of 1 ml to 10 ml may be administered with preferred doses using 4 ml of matrix solution.

The formulations of this invention may be used to treat a variety of animal conditions and physical states. These systems have particular application to pain management, namely the treatment and alleviation of pain associated with any disease, condition or physical state.

Without being limited to the specific pain being treated, the preparations of this invention may treat the following nonlimiting locations or sources of pain: abdominal, such as in appendicitis, dysmenorrhea, musculoskeletal, pelvic, peptic ulcer, psychogenic, and urologic; acute; arm; backache; cancer; cardiac (myocardial ischemia); chest; dental; ear; esophageal; eye; face; head; and neck; in fibromyalgia; foot; and leg; heel; ischemic pain such as in myocardial, peripheral arterial, low back, in mitral valve prolapse, in myocardial infarction, myofascial pain syndrome (fibromyalgia, fibromyositis), neck, neuropathic, neurotransmitter abnormality, nociceptive, and nocturnal pain; pelvic; pericardial; in peripheral arterial disease; phantom limb; pleuritic; polyneuropathy; postmastectomy syndrome; postoperative; psychogenic; in pulmonary embolism; in renal disease, such as colic; root avulsions; shoulder; stump; thalamic; in goes; and toothache.

With regard to uses after surgery, the complex may be used following abdominal, cervical, thoracic or cardiac surgery, whereby multiple layers of tissue, as being sewed back together, are treated with the system. Such treatments aid in a patient's recovery by not only avoiding addictive drug use such as a morphine drip, but result in the immediate and long term relief of pain to enable rapid rehabilitation.

When used epidurally, the formulations of this invention have been found to alleviate pain without the loss of motor or sensory functions. This result is completely unexpected and not known with prior epidural nerve blocks. In this way, patients who are treated epidurally experience a remission or loss of the pain during the time period being treated yet maintain substantially all motor and sensory skills and functions.

The phrase "back pain" as used herein refers to all conditions which arise from congenital or traumatic disfunction of physical structures in the back of an animal, such as a human. These include spinal, bone, nerve and muscle origin pain, and include but are not limited to disc damage caused by trauma, disease or congenital defects; degenerative conditions, arthritic disease, accidental injury, nerve impingement such as pinched nerve, inflammatory conditions, neuromuscular diseases, sports injuries and so forth.

Besides chronic and intractable pain where injections of the matrix solution may be required, the present solutions may be used to aid in surgical procedures such as in post surgical pain treatments.

The phrase "surgical procedure" refers to those internal and external procedures where a physician desires to block sensory effects prior to, during or after performing surgical procedures. While being virtually unlimited in scope as in the procedures which can be performed, exemplary external procedures include: cosmetic surgery, hair transplant surgery, procedures for trauma wounds, such as knife and bullet wounds, accidental lacerations, removal of skin growths such as warts, moles and benign or malignant growths, hemorrhoidal removal or other treatments requiring anesthesia. Furthermore, the inventive compositions are able to provide sustained anesthesia effects when used prior to, during or after internal surgical procedures. Again, without being limited to specific surgical procedures, exemplary procedures would involve abdominal, cervical, thoracic or cardiac surgery.

With regard to uses after surgery, the solutions may be used following abdominal, cervical, thoracic or cardiac surgery, whereby multiple layers of tissue are treated with the system, such as during or after the surgical procedure. Such treatments aid in a patient's recovery by not only avoiding addictive drug use such as a morphine drip, but result in the immediate and long term relief of pain to enable rapid rehabilitation.

It has also been unexpectedly found that when the system is administered in a repetitive manner, once the effects of the active drug are reduced in intensity or effectiveness, such repeat treatments may result in a synergistic effect by enhancing the initial term of relief to a period which exceeds the initial time of relief. This is also experienced on subsequent treatments. In this way, the present formulations are able to extend relief or treatment from normally several hours to at least 12 to 24 hours to several days of relief. The use of repeat injections thus enhances drug release which significantly reduces drug dependence. It also results in the relief of continued tissue damage and may even assist in tissue repaid.

Regardless of the route of administration elected, the formulations of the present invention are formulated into pharmaceutically acceptable dosage forms by conventional methods known in the pharmaceutical art.

As discussed above, an effective but nontoxic amount of the system is employed in treatment. The dose regime for administering drugs or treating various conditions, such as pain as described above, is selected in accordance with a variety of factors including the type, age, weight, sex, and medical condition of the subject, the severity of the pain, the route of administration and the particular complex or combination of drugs employed. Determination of the proper dose for a particular situation is within the skill of the art. Generally, treatment is initiated with smaller dosages which are less than the optimum dose of the compound. Thereafter, the dose is increased by small increments until the optimum effect under the circumstances is reached. For convenience, the total daily dosage may be divided and administered in portions during the day if desired. Generally, amounts of drug may vary from 0.0001% to about 50% by weight of the system when using injections having 2 to 20 ml concentrations and preferably 3 to 10 ml injectable amounts.

Besides treatment of pain, the aqueous system of this invention may be used to treat drug addiction by aiding in the administration of reduced levels of narcotics to addicts as well as using reduced frequency of administration in detoxification and maintenance programs.

In this regard, it should be noted that the withdrawal syndrome from an opioid generally includes symptoms and signs opposite to the drug's pharmacologic effects (e.g., CNS hyperactivity). The severity of the withdrawal syndrome increases with the size of the opioid dose and the duration of dependence. Symptoms begin to appear as early as 4 to 6 hours after withdrawal and reach a peak within 36 to 72 hours for heroin. The initial anxiety and craving for the drug are followed by other symptoms increasing in severity and intensity. A reliable early sign of abstinence is an increased resting respiratory rate, that is greater than 16/min. usually accompanied by yawning, perspiration, lacrimation, and rhinorrhea. Other symptoms include mydriasis, piloerection ("gooseflesh"), tremors, muscle twitching, hot and cold flashes, aching muscles, and anorexia. The withdrawal syndrome in persons who have been taking methadone develops more slowly and overtly less severe than heroin withdrawal, although users may perceive it as worse.

Currently, methadone substitution is the preferred method of opioid withdrawal. Methadone is given orally in the smallest amount that will prevent severe signs of withdrawal but not necessarily all signs. Close observation of the patient is important because the patient's subjective symptoms are unreliable. Many of the symptoms of withdrawal can be mimicked by anxiety states. Generally, 20 mg/day of methadone will block the symptoms of severe withdrawal. Higher doses should be given only on direct observation of the physical signs of withdrawal, since addicts are unreliable in reporting the size of their habits. Doses of 25 to 45 mg can produce unconsciousness if the person has not developed tolerance for heroin or methadone. Once a suppressing dose has been established, it should be reduced progressively by not more than 20% each day. Patients commonly become emotionally upset and frequently request additional medication. Chloral hydrate 500 to 1000 mg may be given orally for several nights to improve sleep. The acute manifestations of withdrawal usually subside within 7 to 10 days, but patients often complain of weakness, insomnia, and a severe pervasive anxiety for several months. Minor metabolic and physiologic effects of withdrawal may persist for up to six months. Conventional procedures treat heroin withdrawal with oral methadone, but the usual low-grade level of dependence can be treated with propoxyphene napsylate or even benzodiazepines, which are not cross-tolerant to opioids. These difficulties would be overcome with use of the present systems.

Unlike methadone, the central α-adrenergic drug clonidine can halt essentially all signs of opioid withdrawal. This probably relates to diminution of central adrenergic outflow secondary to stimulation of central receptors (the same mechanism by which clonidine lowers BP). This theory supports the importance of central adrenergic discharge in the evolution of the opioid withdrawal syndrome. However, clonidine is not a benign drug. Besides causing hypotension and drowsiness, its withdrawal may precipitate restlessness, insomnia, irritability, tachycardia, and headache. Its overall contribution to therapy is minor. Withdrawal is not a difficult problem for patient or clinician; abstinence, which clonidine does not aid, is. The present system would overcome this difficulty.

Experiments with L-acetyl α-methadol (LAAM), a longer-acting synthetic opioid, give hope of help to some addicts and of removing the problem of expensive daily client visits or take-home medication, which ensures some diversion. The culture's loss of faith in methadone maintenance or at least the lessening commitment of public money has diminished the number of treatment facilities and the amount of research given to LAAM.

Unlike the opioids, dependence on anxiolytic and hypnotic drugs raises other difficulties.

Barbiturates and ethanol are strikingly similar in their syndromes of dependence, withdrawal, and chronic intoxication. When intake is reduced below a critical level, a self-limited abstinence syndrome ensures. Symptoms of withdrawal from barbiturates and other sedative-hypnotics can be suppressed completely with a barbiturate. Tolerance develops irregularly and incompletely so that considerable behavioral disturbances and psychotoxicity persist, depending on the drug's pharmacodynamic effects. Some mutual but incomplete cross-tolerance exists between alcohol and the barbiturates as well as the nonbarbiturate sedative-hypnotics, including benzodiazepines.

In susceptible patients, psychologic dependence on the drug may develop rapidly; and, after only a few weeks, attempts to discontinue it exacerbate any initial insomnia and result in restlessness, disturbing dreams, frequent awakening, and feelings of tension in the early morning. The extent of physical dependence is related to dose and the length of time that the drug has been taken; e.g., pentobarbital 200 mg/day may be ingested for many months without significant tolerance developing; 300 mg/day may induce an abstinence syndrome on terminating medication if ingested for more than three moths; and 500 to 600 mg/day may provoke an abstinence syndrome after one month.

The standard procedure for treating dependence on depressants, particularly barbiturates, is to reintoxicate the patient and then withdraw the drug on a strict schedule, being alert for signs of marked withdrawal. Before beginning withdrawal, one can evaluate sedative tolerance with a test dose of pentobarbital 200 mg orally given to the nonintoxicated, fasting patient; 1 to 2 hours later this test dose produces drowsiness or sleep with response to arousal in individuals with no tolerance to pentobarbital. Patients with intermediate levels of tolerance may show some impairment, whereas patients tolerant to 900 mg or more show no signs of intoxication. If the 200-mg dose has no effect, the tolerance level can be determined by repeating the test every 3 to 4 hours with a larger dose. Severe anxiety or agitation may increase the patient's tolerance. Once

The following examples are illustrative of preferred embodiments of the invention and are not to be construed as limiting the invention thereto. All polymer molecular weights are mean average molecular weights. All percentages are based on the percent by weight of the final delivery system or formulation prepared unless otherwise indicated and all totals equal 100% by weight.

### Example 1

This example demonstrates the formation of an anaesthetic preparation which produces long-acting anaesthesia either when injected epidurally, or intramuscularly.

| **MATERIALS** | |
|---|---|
| Bupivacaine | 0.65% |
| Hydroxyethyl cellulose (HEC) | 1.1% |
| Hyaluronate Sodium (HA) | 1.0% |
| Sterile Water | Q.S. |
| Batch Size | 1000 ml |

Into a sterilized glass vessel is added 500ml of the sterile water which is stirred at 400-600 rpms. Slowly add 10 grams of HA having a molecular weight or around 700,000 to 775,000 and a purity described previously.

Allow to stir for 10-20 hours until all the HA Polymer has dissolved into the water and a crystal clear viscous solution has formed.

Prepare a 1.1% solution of HEC by adding 11 grams of the solid material, under aseptic conditions to 275 ml of sterile water. Allow to dissolve for 1 to 2 hours while stirring. Add the HEC solution to the HA solution and mix for 2 to 4 hours at 400-600 rpms until a homogenous solution is produced.

Dissolve 6.5 grams of bupivacaine (bupivacaine) in 225ml of the sterile water and allow to mix for 1-2 hours at 200-400 rpms. Slowly add the bupivacaine solution to the HA/HEC homogenous mixture and mix for 2-4 hours at 400-600 rpms.

The resulting product is a clear solution which should be free of air bubbles.

Using aseptic techniques, the solution is then filled into suitable vials or ampules for use and stored.

### Example 2

The procedure of Example 1 was repeated except that the bupivacaine was replaced with 2% lidocane. The resulting product was a clear solution free of air bubbles.

### Example 3

The procedure of Example 1 was repeated except that the bupivacaine was replaced with 10% adenosine-5-monophosphate sodium. The resulting product was a clear solution free of air bubbles.

### Example 4

The procedure of Example 1 was repeated except that the bupivacaine was replaced with 20 to 80 mg/ml methyl prednesolone. The resulting product was a homogenous and misicable suspension.

### Example 5

The procedure of Example 1 was repeated except that the bupivacaine was replaced with various vitamins, namely 10 milligrams B₁, 15 micrograms folic acid, 1000 micrograms Vitamin B₁₂, or 2 milligrams each of Vitamin B₂ and B₆. The resulting product was a clear solution of free air bubbles.

### Example 6

This example demonstrates the use *in vivo* of the Example 1 preparation with various patients suffering from chronic pain.

### Run A

A 51-year old man has complained of pain in the right temple (this had been present for 15 years), pain in the neck; and generalized headaches.

In 1979, Mr. A was struck in the right temporoparietal area by a heavy machine while at work. Afterwards he developed severe pain in the right temporoparietal area. This gradually spread to involve the whole right side of his head. The pains became more and more frequent until they became constant. In addition he has in the last few years developed ongoing neck pain and generalized headaches.

### Physical Examination

Physical examination has revealed tenderness in the right temporoparietal area as well as in the right supraorbital area and also in the right occipital area. he also has tenderness of the right facet joints and less so on the left. Investigations have included:
1. CT scans of the brain
2. X-rays of the head
3. X-rays of the neck
4. Facet diagnostic blocks of the cervical spin
5. CT myelogram of the cervical spin

### Patient A's diagnosis is as follows:

1. Damage of the peripheral nerves around the periosteum in the right temporoparietal area. (Site of injury headache)
2. Facet joint disease of the cervical spin
3. Recent cervical disc herniation

### Patient A has undergone the following treatments that provided up to 1 day of relief;

1. Injections with local anesthetic and occasionally with steroids of the right supraorbital and temporoparietal area
2. Occipital blocks
3. Paravertebral blocks of the cervical facet joints
In general, the tempoproparietal blocks have provided about one day of relief.

### Treatment with the Example 1 Formulation

Injections were carried out in the right temporal parietal area using about 8 cc of the long-acting bupivacaine formulation.

In addition one injection was made into the paracervical muscle.

### RESULT

The patient obtained about five days of good relief in the temporal parietal area, as well in the trigger point areas.

### SIDE-EFFECTS

The injection of this amount of bupivacaine in a conventional injectable form would have resulted in the onset of pain, inflammation and soreness at the injection site. Relief of pain would have been observed over four to eight hours before reoccurring.

### RUN B

This 43-year old lady is complaining of headaches; neck aches; pain in the mid-thoracic area; low back pain; pain going down the legs; pain going down the arms. She has had these for about ten years.

Ms. B started to have pains in her low back and neck with headaches following a motor vehicle accident in 1978. Following this she was involved in six other motor vehicle accidents. Over the years and with successive accidents, her symptoms increased until she had pains as noted above. Basically she is in pain all the time with pain throughout her body.

Physical examination has shown marked spasm of the whole of the paraspinal musculature from the nuchal line down to the sacrum. The sacroiliac joints were fixed. However, reflexes of the upper and lower limbs were normal indicating that there was no real radiculopathy.

### Patient has undergone the following investigations:

1. X-rays of the cerival and lumbar spines showed some degenerative changes.
2. Her recent CT Myelograms of the lumbar and cervical spines did not reveal any disc herniations.
3. Facet diagnostic blocks of the lumbar and cerivcal spines were equivocal as to whether there was significant facet disease causing the pain or not.

### Patient has undergone the following unsuccessful treatments:

1. Analgesics and especially Fiorinal daily
2. Occasional muscle relaxants
3. Sedatives
4. She was turned down for a psychologically oriented rehabilitation program because it was felt that her problems were too severe and that she would not benefit.
5. Trigger point injections of the paraspinal muscles of the neck, thoracic spin and lumbar spine produced some temporary relief including some relief off the pain down the legs and arms.
6. Occipital blocks produced some temporary relief of the headaches.

The relief by local anesthetic blockades lasted one two days.

### Treatment with the inventive formulation of Example 1:

In the past month the patient was tried on long-acting bupivacaine. The injections were carried out into trigger point areas of the lumbar paravertebral musculature and glueteal musculature.

### RESULT

The patient received about five days of good relief with these trigger point injections.

### SIDE-EFFECTS

With the long-acting Bupivacaine, it was again noted that there was less post-injection pain than the regular Bupivacaine. Furthermore there was no redness at the injection site. Furthermore, the post-injection irritability and general side-effects were less than is experienced with the use of regular Bupivacaine.

### RUN C

This 49-year old lady is complaining of constant neck ache; constant supraorbital pain; pain in the trapezii; mood, memory, concentration disturbances; uncontrolled weeping. She has had these since 1987.

Ms. C was well and active, working as an assistant school principal until 1987 when she was involved in a minor motor vehicle accident. Following this she developed the above-noted symptoms.

She has undergone fact diagnostic blocks which were positive and so indicated facet damage. She underwent facet joint rhizolysis which relieved the pain in her neck and supraorbital areas for about eight months, but then the pains returned. This was presumably to regeneration of the fact nerves.

She has been investigated by neuropsychologists because of her mood, memory and concentration and sleep disturbances. She appears to ahe a combination of minor head injury and post-traumatic emotional disorder. She has not been able to work for several years and this has contributed to a feeling of guilt and worthlessness.

The paracervical muscles are tender and swollen and the facet joints from C2 to C6 are tender bilaterally. The supraorbital nerves are tender to 3+.

### Patient C has undergone the following investigations:

1. Facet diagnostic blocks which were positive
2. CT myelogram which showed a disc herniation at C5-6
3. CT scan of the brain which was normal
4. SPECT scan of the brain which was normal

### Patient C's diagnosis is as follows:

1. Facet damage of the cervical spine causing the headaches including the frontal headaches
2. Cervical disc herniation
3. Post-traumatic emotional disorder
4. Possible minor head injury

### Patient C has undergone the following unsuccessful treatments:

1. Antidepressants
2. Hypnotics for sleep
3. Analgesics for pain
4. Comprehensive psychotherapy
5. Occipital and supraorbital nerve blocks with trigger point injections of the paracervical muscles. These have produced about two days of relief after each block.

### Treatment with the inventive preparation of Example 1:

In the past month this patient was tried on long-acting Bupivacaine. The injections were carried out into the paracervical and trapezius musculature. As well as in the occipital nerves. In addition, supraorbital blocks were done.

### RESULT

The patient received about four to five days of good pain relief in the paracervical musculature, this cut down a lot of the occipital pain. In addition, the supraorbital blockade with long-acting Bupivacaine provided about 4 days of relief. In the past we had used regular Bupivacaine and this provided about 12 hours to one day of relief only.

Once again it was noted that there was no swelling or redness or sense of irritability such as post-injection pain with the present long-acting Bupivacaine formulations.

### Example 7

This example demonstrates the formation of a preparation which produces long-acting analgesia when injected intramuscularly.

| **MATERIALS** | |
|---|---|
| Dilaudid (hydromorphone hydrochloride) | 15 mg |
| Hydroxyethyl cellulose (HEC) | 1.25% |
| Hyaluronate Sodium (HA) | 1.37% |
| Sterile Water | Q.S. |
| Batch Size | 1000 ml |

Into a sterilized glass vessel is added 500 ml of the sterile water which is stirred at 400-600 rpms. Slowly add 13.7 grams of HA having a molecular weight of around 700,000 to 775,000 and a purity described previously.

Allow to stir for 10-20 hours until all the HA polymer has dissolved into the water and a crystal clear viscous solution has formed.

Prepare a 1.25% solution of HEC by adding 12.5 grams of the solid material, under aseptic conditions to 275 ml of sterile water. Allow to dissolve for 1 to 2 hours while stirring. Add the HEC solution to the HA solution and mix for 2 to 4 hours at 490-600 rpms until a homogenous solution is produced. Using aseptic techniques, the solution is then filled into suitable vials or ampules and heat sterilized at 120° C for 20 minutes. The vials or ampules are then ready for use or storage.

Dissolve 15 mg of Dilaudid in 225 ml of the sterile water and allow to mix for 1-2 hours at 200-400 rpms. Alternatively, commercially available solutions of Dilaudid may be used, such as Dilaudid regular containing 2 milligrams drug per milliliter ampule or Dilaudid HP containing 10 milligrams drug per milliliter ampule. Slowly add the Dilaudid solution to 4 ml of the HA/HEC homogenous solution and mix rapidly.

The resulting product is a clear solution which is free of air bubbles and ready for use.

### Example 8

The procedure of Example 7 was repeated except that morphine sulfate was used instead of Dilaudid. The morphine sulfate is provided as a solution blended with the matrix solution and may contain 50 mg drug in 1 ml solution blended with 4 ml matrix as well as 100 mg drug in 1 ml solution blended with 4 ml matrix solution.

### Example 9

The procedure of Example 7 was repeated except that Demerol was used instead of Dilaudid. The Demerol may be conveniently provided in 5 ml ampules containing 50 mg drug. As such doses of 150 mg may be prepared by blending three (3) 5 ml ampules blended with 4 ml of matrix solution. The resulting solution was a clear solution which was free of air bubbles and ready for use.

### Example 10

This example demonstrates the in vivo use of the Example 7 preparation with various patients suffering from chronic pain.

### Run A

### MAIN COMPLAINT:

This 48-year-old man is complaining of right-sided cluster headaches which are continuous.

### HISTORY:

Mr. T. started to develop cluster headaches about 13 years ago. Initially these were on an occasional basis which became gradually more severe and also more frequent. About six years ago, they became constant, and the patient had severe constant pain which almost totally incapacitated him. He has contemplated suicide. The pain is always mainly behind the right eye. This is a severe pain rated as 10/10. It is also in the right occiput and travels like a rod all the way to behind the right eye. It is associated with tenderness of the right eye lid and lacrimation also rhinorrhea. In going over his early history, it was found that he had his first headache at age 11. This occurred when he fell over the front of the handle bars of his bicycle and landed on his chin. At that time, he developed a headache which was exactly like the clusters he has now. This went away after a few days. Following this, he was involved in two minor motor vehicle accidents, and again on each occasion he developed right-sided cluster headaches which eventually cleared up.

### PHYSICAL EXAMINATION:

This reveals a thin man who is somewhat emaciated because of his loss of appetite and severe pain. The right eye lid is constantly drooping, and there is frequent lacrimation from the right eye. There is marked tenderness of the right greater occipital nerve, and less tenderness of the left greater occipital nerve. The right facet joints at C2-3, C3-4 are also tender. Facet diagnostic blocks did not relieve the frontal pain. Occipital blocks have relieved the pain going from the occiput through toward the right eye, but did not entirely relieve the pain in the right eye. The only way this has been relieved is when the right infraorbital or retro-orbital blocks have been done in addition to the occipital blocks.

### TREATMENT COURSE:

He has been having weekly blocks of the greater occipital nerve and infratrochlear nerve for two years now. This provide up to 36 hours of relief. The rest of the time, he has such severe pain that he would be totally incapacitated without narcotics. For the past nine months, he has been prescribed Dilaudid 4 mg every four hours. This has to be taken with Benadryl, otherwise

### USE OF LONG-ACTING POLYMER WITH DILAUDID:

The regular Dilaudid had to be given at least six times a day. However, when Dilaudid 20 mg was mixed with polymer solution of Example 7, about 12 to 16 hours of relief could be obtained. The quality of relief is very good, and he is able to work as a movie director as long as he has pain relief. Previously, he was incapacitated from work by the pain. As indicated, with the plain Dilaudid injection he gets about 2 hours of fairly good relief, but with the polymer solution and Dilaudid he gets 12 to 16 hours of excellent relief. During this time, he is not sleepy or uncomfortable and feels reasonably normal.

### Run B

### MAIN COMPLAINT:

This 38-year-old woman has complained of pain in the left cervico-occipital temporal area for about ten years.

### HISTORY:

In about 1979, Ms. G was abused on a continuing basis by her husband. He would hold her head and hit it against the wall and shake the head. It was at that time that she started to develop left occipitocervical temporal headaches. These were initially infrequent, but in the past ten years they have been almost continuous. She was mistakenly diagnosed as having migraine by her previous physicians.

### PHYSICAL EXAMINATION:

This revealed tenderness in the left greater occipital nerve area to 4+, and tenderness of the left facet joints at C2-3 and C3-4. The right greater occipital nerve and facet joints were not especially tender.

### INVESTIGATIONS:

1. X-rays of the head and neck were normal.
2. Facet diagnostic blocks at C2-3 and C3-4 on the left were negative.
3. C2 diagnostic block on the left entirely relieved her pain.
4. Greater occipital nerve blocks on the left have relieved her pain.
The diagnosis was as follows: Damage of the occipitocervical junction possibly the C1-2 facet area. This is causing a left occipital neuralgia.

### TREATMENT:

Occipital nerve blocks initially provided 2 to 3 days of relief, but more recently they have only provided one day of relief at a time. She has been getting occipital blocks on a weekly basis which relieved her pain for a couple of days at a time, but more recently one day at a time. In between she has been getting Percocet. However, in the last few months she could not take Percocet because of the gastrointestinal disturbance. She was then put on Dilaudid 4 mg every four hours. This made her very weak, and she could not function after it, and she found she had to take it at least every four hours and sometimes every three hours.

### USE OF LONG-ACTING POLYMER WITH DILAUDID:

When 10 mg of Dilaudid was administered with the polymer solution, the pain relief was 16 to 18 hours with each injection. This produced a smooth degree of relief, and she was not nearly as tired or weak as when she took the Dilaudid without polymer solution.

### Run C

### MAIN COMPLAINT:

This 48-year-old nurse is complaining of severe constant headaches and neck aches. She has had these for about eight years.

### HISTORY:

Ms. W. was involved in a motor vehicle accident in 1988. Following this, she was unconscious for a few moments. The next day, she started to develop severe neck aches and headaches which she had never experienced before, and these have not improved over time. Currently she has almost constant severe neck aches which radiate up the back of the head and involve the whole head. Often these are of a throbbing nature. She has had numerous nerve blocks and pain killers over the years to keep her going. Without these treatments, she would be totally incapacitated.

### PHYSICAL EXAMINATION:

Examination has revealed marked limitation of movement of the

### PHYSICAL EXAMINATION:

Examination has revealed marked limitation of movement of the neck to about 30% of normal in both flexion and extension and rotation. The neck muscles are very tender to palpation, and they are swollen. The occipital nerves are tender to 3-bilaterally, and the face joints are exquisitely tender bilaterally from C2 to C6.

### INVESTIGATIONS:

1. X-rays of the neck showed degenerative changes.
2. MRI of the neck has shown disc herniations.

### TREATMENTS:

As noted, she has been receiving occipital nerve blocks with analgesics for several years, but she has required increasingly frequent nerve blocks and increasing doses of analgesics. Recently, she had been using fentanyl patches 50 µ/hr. These worked well initially, but ceased to be effective after a few weeks. She was then given Dilaudid 4 mg every four hours by intramuscular injection. She would obtain about two hours of good relief with some degree of relief in the first hour before the injection reached its peak level, and begin partial relief toward the end of the four hours. As noted, there were only two hours of good relief each time.

### USE OF LONG-ACTING POLYMER WITH DILAUDID:

She was then given Dilaudid blended with polymer solution as set forth in Example 7 wherein she experienced about 18 hours of good relief. During this time, she was able to function well and could carry out her business functions entirely comfortably without any nausea or tiredness. She found the use of this drug to be very comfortable, and she was able to function normally.

**TABLE I**

| EQUIANALGESIC DOSES OF OPIOID ANALGESICS FOR SEVERE PAIN* | | |
|---|---|---|
| Drug | IM (mg) | Oral (mg) |
| Morphine | 10 | 60† |
| Oxymorphone | 1 | |
| Hydromorphone | 1.5 | 7.5 |
| Levorphanol | 2 | 4 |
| Methadone | 10 | 20 |
| Oxycodone | 15 | 30 |
| Meperidine | 75 | 300 |
| Codeine | 130 | 200 |
| Pentazocine | 60 | 180 |
| Nalbuphine | 10 | - |
| Butorphanol | 2 | - |

| | | |
|---|---|---|
| * Equivalences are based on single-dose studies. | | |

**TABLE II**

| Drug | Doses Producing Dependence (mg/day) | Time Necessary to Produce Dependence (days) | Dosage Equivalent to 30 mg Phenobarbital (mg) |
|---|---|---|---|
| Secobarbital | 500-600 | 30 | 100 |
| Pentobarbital ("yellow jackets") | 500-600 | 30 | 100 |
| Amobarbital ("blues") | 500-600 | 30 | 100 |
| Amobarbital-secobarbital combination ("rainbows") | 500-600 | 30 | 100 |
| Gluethimide | 1250-1500 | 60 | 500 |
| Methyprylon | 1200-1500 | 60 | 300 |
| Ethchlorvynol | 1500-2000 | 60 | 500 |
| Meprobamate | 2000-2400 | 60 | 400 |
| Chlordiazepoxide | 200-300 | 60 | 25 |
| Diazepam | 60-100 | 40 | 10 |
| Methaqualone | 1800-2400 | 30 | 300 |
| Chloral hydrate | 2000-2500 | 30 | 500 |

## Claims

1. A sustained release delivery system, which comprises a polymer matrix solubilized or suspended in a liquid medium, wherein the polymer matrix is composed of a salt of hyaluronic acid blended with a non-ionic polymer selected from hydroxyethyl cellulose, hydroxypropyl cellulose and mixtures thereof.

2. The system of claim 1, wherein the salt of hyaluronic acid has a mean average molecular weight below about 800,000.

3. The system of claim 2, wherein the hyaluronic acid is in the form of its sodium salt and has a mean average molecular weight of from about 650,000 to about 800,000.

4. The system of claim 2 or claim 3, wherein the hyaluronic acid is in the form of its sodium salt and has a mean average molecular weight of from about 700,000 to about 775,000.

5. The system of claim 3 or claim 4, wherein the hyaluronic acid has a sulphated ash content below about 15%, and a protein content below about 5%.

6. A system according to any preceding claim, wherein the molar ratio of the salt of hyaluronic acid to the non-ionic polymer is in the range of from 1:0.5 to 2.

7. The system of any preceding claim, wherein the hyaluronic acid salt is its sodium, calcium, potassium or magnesium salt.

8. The system of any preceding claim, wherein the non-ionic polymer is hydroxyethyl cellulose.

9. The system of any preceding claim, wherein the molar ratio of the salt of hyaluronic acid to the non-ionic polymer is in the range of from 1:0.8 to 1.5.

10. The system of any preceding claim, wherein the salt of hyaluronic acid is present in an amount of from about 0.1% to about 3.0% by weight.

11. A system according to any preceding claim, in the form of a solution.

12. A system according to any preceding claim, in a form suitable for injection.

13. A system according to any of claims 1 to 10, in the form of gel.

14. A system according to any of claims 1 to 10 or 13, in a form suitable for topical application.

15. A long-acting drug composition, which comprises an active drug dispersed, solubilized or suspended in a polymer matrix of a sustained release delivery system according to any of claims 1 to 14.

16. A composition according to claim 15, wherein the active drug comprises a water-soluble solid and wherein the polymer matrix is solubilized in an aqueous medium.

17. A long-acting drug composition, which comprises an active drug and a polymer matrix of a sustained release delivery system according to any of claims 1 to 14, wherein the liquid medium is an aqueous medium and wherein the drug and polymer matrix are separately contained, whereby the drug maybe blended or mixed with the polymer matrix immediately prior to use.

18. A composition according to any of claims 15 to 17, wherein the active drug is selected from anaesthetics, analgesics and antagonists, anti-histamines, antitussives, expectorants, decongestants, antibiotics, cardiovascular preparations, metal salts, minerals, immunomodulators, immunosuppressives, steroids and hormones, vitamins and veterinary formulations.

19. A method for making a sustained release delivery system according to any of claims 1 to 14, which method comprises blending an aqueous solution of the salt of hyaluronic acid with an aqueous solution of the non-ionic polymer to form the polymer matrix.

20. The method of claim 19, wherein the solution of the salt of hyaluronic acid is added to the non-ionic polymer solution.

21. The method of claim 19, wherein the non-ionic polymer solution is added to the solution of the salt of hyaluronic acid.

22. The method of any of claims 19 to 21, wherein the solution of the salt of hyaluronic acid and/or the non-ionic polymer solution is purified prior to blending.

23. A method for making a long-acting analgesic or antagonist injectable drug solution, which method comprises preparing a polymer matrix comprising a salt of hyaluronic acid having a mean average molecular weight between about 650,000 and 800,000 and a non-ionic polymer, wherein the molar ratio of the salt of hyaluronic acid to the non-ionic polymer is 1:0.5 to 2 and the salt of hyaluronic acid is present in amounts of about 0.1% to about 2.0% by weight; solubilizing within the polymer matrix a water-soluble analgesic or antagonist drug by blending the drug with the polymer matrix solution; and recovering the resulting solution.

24. The method of claim 23, wherein the drug is a water-soluble solid which is mixed into the polymer matrix solution prior to use.

25. The method of claim 23, wherein the drug is in the form of a solution prior to mixing with the polymer matrix solution.

26. The use of a system according to any of claims 1 to 14 in the preparation of a long-acting medicament for treating acute, chronic or intractable diseases or conditions.

27. The use according to claim 26, wherein the medicament is suitable for injection or for topical administration.

28. The use according to claim 26 or 27, wherein the hyaluronic acid is in the form of its sodium salt and has a mean average molecular weight in the range of from about 650,000 to about 800,000; a sulphated ash content below about 15%; a protein content below about 5%; and purity of at least 98%.

29. The use according to any of claims 26 to 28, wherein the medicament is administrable epidurally, sub-cutaneously, epidermally or intra-muscularly.

30. The use according to any of claims 26 to 29, wherein the system enables therapeutically effective levels of the active drug to be released from the medicament for at least 12 hours.

31. The use according to any of claims 26 to 30, for the preparation of a medicament for treating or alleviating pain, wherein the system enables therapeutically effective levels of the drug to be released from the medicament for periods of time of at least 18 hours.

32. The use according to any of claims 26 to 31, wherein the condition to be treated is chronic, intractable or sympathetically-mediated pain.

33. The use according to any of claims 26 to 32, wherein the medicament is for alleviating pain without significantly modifying motor or sensory functions.

34. The use according to any of claims 26 to 33, wherein the condition to be treated is back pain.

35. The use according to any of claims 26 to 34, wherein the medicament is for administration during or after abdominal, cervical, thoracic or cardiac surgery, for treating post-operative pain or for use post-amputation.

36. The use according to any of claims 26 to 34, wherein the medicament is for treating pain associated with or caused by abnormal cell growth, cancer, tumour mass, arthritis, sickle cell disease, haemophilia, pinched nerve, damaged nerve or migraine.

37. The use according to any of claims 26 to 30, wherein the medicament is for treating drug addiction.

## Patentansprüche

1. Verabreichungssystem mit verzögerter Freisetzung, das eine in einem flüssigen Medium solubilisierte oder suspendierte Polymermatrix umfasst, worin die Polymermatrix gebildet ist aus einem Hyaluronsäuresalz vermengt mit einem nichtionischen Polymer ausgewählt aus Hydroxyethylcellulose, Hydroxypropylcellulose und Mischungen davon.

2. System nach Anspruch 1, worin das Hyaluronsäuresalz ein durchschnittliches mittleres Molekulargewicht unter ungefähr 800000 aufweist.

3. System nach Anspruch 2, worin die Hyaluronsäure in Form ihres Natriumsalzes vorliegt und ein durchschnittliches mittleres Molekulargewicht von ungefähr 650000 bis ungefähr 800000 aufweist.

4. System nach Anspruch 2 oder 3, worin die Hyaluronsäure in Form ihres Natriumsalzes vorliegt und ein durchschnittliches mittleres Molekulargewicht von ungefähr 700000 bis ungefähr 775000 aufweist.

5. System nach Anspruch 3 oder 4, worin die Hyaluronsäure einen Sulfataschegehalt unter ungefähr 15 % aufweist, und einen Proteingehalt unter ungefähr 5 %.

6. System nach einem der vorhergehenden Ansprüche, worin das Molverhältnis des Hyaluronsäuresalzes zum nichtionischen Polymer im Bereich von 1 : 0,5 bis 2 liegt.

7. System nach einem der vorhergehenden Ansprüche, worin das Hyaluronsäuresalz ein Natrium-, Calcium-, Kalium- oder Magnesiumsalz ist.

8. System nach einem der vorhergehenden Ansprüche, worin das nichtionische Polymer Hydroxyethylcellulose ist.

9. System nach einem der vorhergehenden Ansprüche, worin das Molverhältnis des Hyaluronsäuresalzes zum nichtionischen Polymer im Bereich von 1 : 0,8 bis 1,5 liegt.

10. System nach einem der vorhergehenden Ansprüche, worin das Hyaluronsäuresalz in einer Menge von ungefähr 0,1 Gew.-% bis ungefähr 3,0 Gew.-% vorhanden ist.

11. System nach einem der vorhergehenden Ansprüche, in Form einer Lösung.

12. System nach einem der vorhergehenden Ansprüche, in einer für eine Injektion geeigneten Form.

13. System nach einem der Ansprüche 1 bis 10, in Form eines Gels.

14. System nach einem der Ansprüche 1 bis 10 oder 13, in einer für topische Anwendung geeigneten Form.

15. Langwirkende Arzneizusammensetzung, die einen aktiven Arzneistoff in einer Polymermatrix eines Verabreichungssystems mit verzögerter Freisetzung nach einem der Ansprüche 1 bis 14 dispergiert, solubilisiert oder suspendiert umfasst.

16. Zusammensetzung nach Anspruch 15, worin der aktive Arzneistoff einen wasserlöslichen Feststoff umfasst und worin die Polymermatrix in einem wässrigen Medium solubilisiert ist.

17. Langwirkende Arzneizusammensetzung, die einen aktiven Arzneistoff und eine Polymermatrix eines Verabreichungssystems mit verzögerter Freisetzung nach einem der Ansprüche 1 bis 14 umfasst, worin das flüssige Medium ein wässriges Medium ist und worin der Arzneistoff und die Polymermatrix separat enthalten sind, wodurch der Arzneistoff mit der Polymermatrix unmittelbar vor Verwendung vermengt oder vermischt werden kann.

18. Zusammensetzung nach Anspruch 15 bis 17, worin der aktive Arzneistoff ausgewählt ist aus Anästhetika, Analgetika und Antagonisten, Antihistaminika, Antitussiva, Expektorantia, Dekongestionsmitteln, Antibiotika, kardiovaskulären Präparaten, Metallsalzen, Mineralstoffen, Immunmodulatoren, Immunsuppressiva, Steroiden und Hormonen, Vitaminen und veterinären Formulierungen.

19. Verfahren zur Herstellung eines Verabreichungssystems mit verzögerter Freisetzung nach einem der Ansprüche 1 bis 14, welches Verfahren Vermengen einer wässrigen Lösung des Hyaluronsäuresalzes mit einer wässrigen Lösung des nichtionischen Polymers umfasst, um die Polymermatrix auszubilden.

20. Verfahren nach Anspruch 19, worin die Lösung des Hyaluronsäuresalzes der Lösung des nichtionischen Polymers zugesetzt wird.

21. Verfahren nach Anspruch 19, worin die Lösung des nichtionischen Polymers der Lösung des Hyaluronsäuresalzes zugesetzt wird.

22. Verfahren nach einem der Ansprüche 19 bis 21, worin die Lösung des Hyaluronsäuresalzes und/oder die Lösung des nichtionischen Polymers vor dem Vermengen gereinigt wird.

23. Verfahren zur Herstellung einer injizierbaren Arzneistofflösung mit einem langwirkenden Analgetikum oder Antagonisten, welches Verfahren umfasst Herstellen einer Polymermatrix umfassend ein Hyaluronsäuresalz mit einem durchschnittlichen mittleren Molekulargewicht zwischen ungefähr 650000 und 800000 und einem nichtionischen Polymer, worin das Molverhältnis des Hyaluronsäuresalzes zum nichtionischen Polymer im Bereich von 1 : 0,5 bis 2 liegt und das Hyaluronsäuresalz in Mengen von ungefähr 0,1 Gew.-% bis ungefähr 2,0 Gew.-% vorhanden ist; Solubilisieren eines wasserlöslichen Anatgetikum- oder Antagonistenarzneistoffs in der Polymermatrix durch Vermengen des Arzneistoffs mit der Polymermatrixlösung; und Gewinnen der erhaltenen Lösung.

24. Verfahren nach Anspruch 23, worin der Arzneistoff ein wasserlöslicher Feststoff ist, der vor der Verwendung in die Polymermatrixlösung gemischt wird.

25. Verfahren nach Anspruch 23, worin der Arzneistoff vor dem Vermischen mit der Polymermatrixlösung in Form einer Lösung vorliegt.

26. Verwendung eines Systems nach einem der Ansprüche 1 bis 14 bei der Herstellung eines langwirkenden Medikaments zur Behandlung von akuten, chronischen oder hartnäckigen Krankheiten oder Zuständen.

27. Verwendung nach Anspruch 26, worin das Medikament zur Injektion oder für topische Verabreichung geeignet ist.

28. Verwendung nach Anspruch 26 oder 27, worin die Hyaluronsäure in Form ihres Natriumsalzes vorliegt und ein durchschnittliches mittleres Molekulargewicht im Bereich von ungefähr 650000 bis ungefähr 800000 aufweist; einen Sulfataschegehalt unter ungefähr 15 %; einen Proteingehalt unter ungefähr 5 %; und eine Reinheit von mindestens 98 %.

29. Verwendung nach einem der Ansprüche 26 bis 28, worin das Medikament epidural, subkutan, epidermal oder intramuskulär verabreichbar ist.

30. Verwendung nach einem der Ansprüche 26 bis 29, worin das System ermöglicht, dass therapeutisch wirksame Mengen des aktiven Arzneistoffs aus dem Medikament über mindestens 12 Stunden freigesetzt werden.

31. Verwendung nach einem der Ansprüche 26 bis 30, zur Herstellung eines Medikaments zur Behandlung oder Linderung von Schmerzen, worin das System ermöglicht, dass therapeutisch wirksame Mengen des Arzneistoffs aus dem Medikament über Zeiträume von mindestens 18 Stunden freigesetzt werden.

32. Verwendung nach einem der Ansprüche 26 bis 31, worin der zu behandelnde Zustand chronische, hartnäckige oder Sympathikus bezogene Schmerzen sind.

33. Verwendung nach einem der Ansprüche 26 bis 32, worin das Medikament zur Linderung von Schmerzen, ohne signifikante Beeinflussung der motorischen oder sensorischen Funktionen vorgesehen ist.

34. Verwendung nach einem der Ansprüche 26 bis 33, worin der zu behandelnde Zustand Rückenschmerzen sind.

35. Verwendung nach einem der Ansprüche 26 bis 34, worin das Medikament zur Verabreichung bei oder nach abdominalen, zervikalen, thorakalen oder kardialen chirurgischen Eingriffen vorgesehen ist, zur Behandlung postoperativer Schmerzen oder zur postamputativen Verwendung.

36. Verwendung nach einem der Ansprüche 26 bis 34, worin das Medikament zur Behandlung von Schmerzen in Verbindung mit oder ausgelöst durch abnormales Zellwachstum, Krebs, Tumormasse, Arthritis, Sichelzellenanämie, Haemophilie, eingeklemmten Nerv, Nervenschädigung oder Migräne vorgesehen ist.

37. Verwendung nach einem der Ansprüche 26 bis 30, worin das Medikament zur Behandlung von Drogenabhängigkeit vorgesehen ist.

## Revendications

1. Système de distribution à libération prolongée, qui comprend une matrice de polymère solubilisée ou en suspension dans un milieu liquide, dans lequel la matrice de polymère est composée d'un sel d'acide hyaluronique mélangé avec un polymère non ionique choisi parmi une hydroxyéthyl cellulose, une hydroxypropyl cellulose et leurs mélanges.

2. Système suivant la revendication 1, dans lequel le sel d'acide hyaluronique a un poids moléculaire moyen en moyenne inférieure à environ 800.000.

3. Système suivant la revendication 2, dans lequel l'acide hyaluronique est sous la forme de son sel de sodium et a un poids moléculaire moyen en moyenne d'environ 650.000 à environ 800.000.

4. Système suivant la revendication 2 ou la revendication 3, dans lequel l'acide hyaluronique est sous la forme de son sel de sodium et a un poids moléculaire moyen en moyenne d'environ 700.000 à environ 775.000.

5. Système suivant la revendication 3 ou la revendication 4, dans lequel l'acide hyaluronique a une teneur en cendres sulfatées inférieure à environ 15%, et une teneur en protéine inférieure à environ 5%.

6. Système suivant l'une quelconque des revendications précédentes, dans lequel le rapport molaire du sel d'acide hyaluronique au polymère non ionique est compris dans la gamme de 1:0,5 à 2.

7. Système suivant l'une quelconque des revendications précédentes, dans lequel le sel d'acide hyaluronique est son sel de sodium, de calcium, de potassium ou de magnésium.

8. Système suivant l'une quelconque des revendications précédentes, dans lequel le polymère non ionique est une hydroxyéthyl cellulose.

9. Système suivant l'une quelconque des revendications précédentes, dans lequel le rapport molaire du sel d'acide hyaluronique au polymère non ionique est compris dans la gamme de 1:0,8 à 1,5.

10. Système suivant l'une quelconque des revendications précédentes, dans lequel le sel d'acide hyaluronique est présent en une quantité d'environ 0,1% à environ 3,0% en poids.

11. Système suivant l'une quelconque des revendications précédentes, sous la forme d'une solution.

12. Système suivant l'une quelconque des revendications précédentes, sous une forme convenable pour injection.

13. Système suivant l'une quelconque des revendications 1 à 10, sous la forme d'un gel.

14. Système suivant l'une quelconque des revendications 1 à 10 ou 13, sous une forme convenable pour une application topique.

15. Composition de médicament à longue durée d'action, qui comprend un médicament actif dispersé, solubilisé ou en suspension dans une matrice de polymère d'un système de distribution à libération prolongée suivant l'une quelconque des revendications 1 à 14.

16. Composition suivant la revendication 15, dans laquelle le médicament actif comprend un solide soluble dans l'eau et dans laquelle la matrice de polymère est solubilisée dans un milieu aqueux.

17. Composition de médicament à longue durée d'action, qui comprend un médicament actif et une matrice de polymère d'un système de distribution à libération prolongée suivant l'une quelconque des revendications 1 à 14, dans laquelle le milieu liquide est un milieu aqueux et dans laquelle le médicament et la matrice de polymère sont contenus de façon séparée, de telle sorte que le médicament peut être mélangé ou mêlé à la matrice de polymère immédiatement avant emploi.

18. Composition suivant l'une quelconque des revendications 15 à 17, dans laquelle le médicament actif est choisi parmi des anesthésiques, des analgésiques et des antagonistes, des antihistaminiques, des antitussifs, des expectorants, des décongestionnants, des antibiotiques, des préparations cardiovasculaires, des sels métalliques, des éléments minéraux, des immunomodulateurs, des immunosuppresseurs, des stéroïdes et des hormones, des vitamines et des formulations vétérinaires.

19. Procédé pour préparer un système de distribution à libération prolongée suivant l'une quelconque des revendications 1 à 14, lequel procédé comprend le mélange d'une solution aqueuse du sel de l'acide hyaluronique avec une solution aqueuse du polymère non ionique pour former la matrice de polymère.

20. Procédé suivant la revendication 19, dans lequel la solution du sel d'acide hyaluronique est ajoutée à la solution de polymère non ionique.

21. Procédé suivant la revendication 19, dans lequel la solution de polymère non ionique est ajoutée à la solution du sel d'acide hyaluronique.

22. Procédé suivant l'une quelconque des revendications 19 à 21, dans lequel la solution du sel d'acide hyaluronique et/ou la solution de polymère non ionique sont purifiées avant le mélange.

23. Procédé pour préparer une solution de médicament injectable antagoniste ou analgésique à action de longue durée, lequel procédé comprend la préparation d'une matrice de polymère comprenant un sel d'acide hyaluronique ayant un poids moléculaire moyen en moyenne compris entre environ 650.000 et 800.000 et un polymère non ionique, dans lequel le rapport molaire du sel d'acide hyaluronique au polymère non ionique est de 1:0,5 à 2 et le sel d'acide hyaluronique est présent en des quantités d'environ 0,1% à environ 2,0% en poids ; la solubilisation à l'intérieur de la matrice de polymère d'un médicament antagoniste ou analgésique soluble dans l'eau par mélange du médicament avec la solution de matrice de polymère ; et la récupération de la solution résultante.

24. Procédé suivant la revendication 23, dans lequel le médicament est un solide soluble dans l'eau qui est mélangé à la solution de matrice de polymère avant utilisation.

25. Procédé suivant la revendication 23, dans lequel le médicament est sous la forme d'une solution avant mélange avec la solution de matrice de polymère.

26. Utilisation d'un système suivant l'une quelconque des revendications 1 à 14 dans la préparation d'un médicament à longue durée d'action pour le traitement d'états ou d'affections aigus, chroniques ou réfractaire.

27. Utilisation suivant la revendication 26, dans laquelle le médicament convient pour l'injection ou l'administration topique.

28. Utilisation suivant la revendication 26 ou 27, dans laquelle l'acide hyaluronique est sous la forme de son sel de sodium et a un poids moléculaire moyen en moyenne compris dans la gamme d'environ 650.000 à environ 800.000 ; une teneur en cendres sulfatées inférieure à environ 15% ; une teneur en protéine inférieure à environ 5% ; et une pureté d'au moins 98%.

29. Utilisation suivant l'une quelconque des revendications 26 à 28, dans laquelle le médicament peut être administré par voie épidurale, sous-cutanée, épidermique ou intramusculaire.

30. Utilisation suivant l'une quelconque des revendications 26 à 29, dans laquelle le système permet la libération de taux efficaces du point de vue thérapeutique du médicament à partir du médicament pendant au moins 12 heures.

31. Utilisation suivant l'une quelconque des revendications 26 à 30, pour la préparation d'un médicament pour le traitement ou le soulagement de la douleur, dans laquelle le système permet la libération de taux efficaces du point de vue thérapeutique du médicament actif à partir du médicament pendant des périodes de temps d'au moins 18 heures.

32. Utilisation suivant l'une quelconque des revendications 26 à 31, dans laquelle l'état à traiter est une douleur chronique, réfractaire ou médiée par les voies sympathiques.

33. Utilisation suivant l'une quelconque des revendications 26 à 32, dans laquelle le médicament est destiné à soulager la douleur sans modifier de façon significative les fonctions motrices ou sensorielles.

34. Utilisation suivant l'une quelconque des revendications 26 à 33, dans laquelle l'état à traiter est une douleur du dos.

35. Utilisation suivant l'une quelconque des revendications 26 à 34, dans laquelle le médicament est destiné à être administré pendant ou après un acte chirurgical abdominal, cervical, thoracique ou cardiaque, pour le traitement de la douleur post-opératoire ou pour une utilisation après amputation.

36. Utilisation suivant l'une quelconque des revendications 26 à 34, dans laquelle le médicament est destiné à traiter une douleur associée ou provoquée par une croissance cellulaire anormale, un cancer, une masse tumorale, de l'arthrite, l'affectation des cellules falciformes, une hémophilie, des névralgies, des névrites ou une migraine.

37. Utilisation suivant l'une quelconque des revendications 26 à 30, dans laquelle le médicament est destiné au traitement d'une pharmacodépendance.
